# EUROPEAN PATENT SPECIFICATION

(11) **EP 4 543 517 B1**
(45) Date of publication and mention of the grant of the patent: **09.09.2026**
(21) Application number: 23735301.6
(22) Date of filing: 26.06.2023
(51) Int. Cl.: A61B 5/085, A61M 16/00, A61M 16/04, A61M 16/08, A61M 16/20, A61B 5/03, A61B 5/08, A61B 5/00

(54) **SYSTEM AND METHOD FOR CHARACTERIZING AN INFLATABLE CUFF**
SYSTEM UND VERFAHREN ZUR CHARAKTERISIERUNG EINER AUFBLASBAREN MANSCHETTE
SYSTÈME ET PROCÉDÉ DE CARACTÉRISATION D'UN BRASSARD GONFLABLE

(30) Priority: 27.06.2022 EP 22181293
(43) Date of publication of application: 30.04.2025
(73) Proprietor: AW Technologies ApS, 9400 Nørre Sundby (DK)
(72) Inventor: CASPER CLEMENT, Borre, 9500 Hobro (DK); BENNEDBÆK, Dennis Schmidt, 9000 Aalborg (DK); HANSEN, Adam Søholm, 9210 Aalborg SØ (DK); HEJSLET, Andreas Achton Mortensen, 9000 Aalborg (DK)
(74) Representative: Høiberg P/S
(86) International application number: PCT/EP2023/067330
(87) International publication number: WO 2024/002973

(56) References cited:
- US-B2- 10 369 312
- US-B2- 9 474 469

## Description

The present disclosure relates to a system for characterizing an inflatable cuff arranged around a tracheal tube for providing breathing support to a patient. The disclosure further relates to method for characterizing an inflatable cuff arranged around a tracheal tube for providing breathing support to a patient.

### Background

As known, ventilated patients, particularly those who are in intensive therapy, are intubated with endotracheal tubes or tracheal tubes. Many tubes have an inflatable cuff to seal the trachea against air leakage and aspiration of gastric contents, blood, secretions, and other fluids while allowing the patient to breathe through the tracheal tube.

The characteristics of such inflatable tube cuffs may deteriorate over time, which can cause leakage around the cuff. The presence of such leakage may decrease the efficiency of the ventilator and may also lead to bacterial aspiration to the lungs, potentially increasing the risk of pneumonia, which may prolong the time that the patient stays in the hospital.

Patients that have pneumonia have a significantly increased length of hospital stay, a directly attributable increased mortality of and increased reintubation rate.

Several techniques are used to monitor and control the cuff pressure over time. For example, a pressure may be maintained above a certain minimum pressure or within a certain pressure range. However, monitoring the pressure of the cuff or maintaining a certain pressure in the cuff does not provide any detailed information about the status of the cuff, possible leakage around the cuff or other issues with the cuff or patient.

The following documents are known in the art, namely US 10 369 312 B2 and US 9 474 469 B2.

### Summary

The invention is defined in the appended claims.

One object of the present disclosure is to provide a system for characterizing ar inflatable cuff arranged around a tracheal tube for providing breathing support to a patient. A first aspect of the disclosure therefore relates to a system for characterizing an inflatable cuff arranged around a tracheal tube for providing breathing support to a patient, the system comprising:
at least one flow unit, such as a pump, in fluid connection with a cuff conduit connected to the inflatable cuff, wherein the at least one flow unit is adapted to fill the inflatable cuff with gas or liquid;
at least one pressure sensor configured to measure a pressure in the inflatable cuff;
at least one flow sensor for measuring a flow of gas or liquid to and/or from the inflatable cuff;
a processing unit for estimating the volume of gas or liquid or a change of the volume of gas or liquid in the inflatable cuff based on measurements of the flow of gas or liquid to and/or from the inflatable cuff, wherein the processing unit is configured to:
   extract a pressure-volume relationship between the measured pressure and the estimated volume of gas or liquid or a change of the volume of gas or liquid in the inflatable cuff, and
compare the pressure-volume relationship against a previously measured or a predetermined reference pressure-volume relationship to characterize the inflatable cuff.

By extracting a pressure-volume relationship between the measured pressure and the estimated volume of gas or liquid or a change of the volume of gas or liquid in the inflatable cuff, and comparing the pressure-volume relationship against a previously measured or a predetermined reference pressure-volume relationship, a change of the pressure-volume relationship over time may be obtained. The pressure-volume relationship may be, for example, a coefficient converting or translating at least one given volume to a corresponding pressure, or converting or translating at least one given pressure to a corresponding volume. The change of the pressure-volume relationship may be used to characterize the inflatable cuff non-invasively.

The characterization of the inflatable cuff may provide a measure of elasticity or of the inflatable cuff, but may also provide information about the structure around the inflatable cuff, usually the trachea. The pressure-volume relationship in a given configuration may be represented by a coefficient converting at least one given volume to a corresponding pressure, or converting at least one given pressure to a corresponding volume. A higher coefficient would normally mean that a higher pressure is required to obtain a given volume. As would be understood by a person skilled in the art, it would, equivalently, be possible to use a coefficient with the opposite meaning. The processing unit may then be used to interpret the change of the pressure-volume relationship. If the coefficient is less than a previously measured coefficient, it would mean that a lower pressure than in the first measurement is required to obtain a given volume. This may indicate a more elastic inflatable cuff. If the change of elasticity exceeds a predefined limit, the system may categorize the inflatable cuff as worn out. The predefined limit is typically not a limit for all inflatable cuffs in all patients be a limit for a specific inflatable cuff, possibly in a specific patient and possibly at a specific time. If the coefficient is higher than a previously measured coefficient, it would mean that a higher pressure than in the first measurement is required to obtain a given volume. Since the inflatable cuff does typically not get more difficult to fill with gas or liquid over time, the change to a higher coefficient may be interpreted as the structure around the inflatable cuff providing external pressure on the inflatable cuff. Hence, in one embodiment the system is further configured to identify a stiffer trachea of the patient as a result of an increase of the coefficient.

The disclosure further relates to a method for characterizing an inflatable cuff arranged around a tracheal tube for providing breathing support to a patient, the method comprising the steps of:
measuring a pressure in the inflatable cuff in the inflated configuration;
measuring a flow of gas to and/or from the inflatable cuff;
estimating the volume of gas or a change of the volume of gas or liquid in the inflatable cuff based on the measurements of the flow of gas to and/or from the inflatable cuff;
extracting a pressure-volume relationship between the measured pressure and the estimated volume of gas or a change of the volume of gas or liquid in the inflatable cuff, and
comparing the pressure-volume relationship against a previously measured or a predetermined reference pressure-volume relationship to characterize the inflatable cuff.

As would be understood by a person skilled in the art, the presently disclosed method for characterizing an inflatable cuff arranged around a tracheal tube for providing breathing support to a patient be performed using any embodiment of the presently disclosed system for characterizing an inflatable cuff arranged around a tracheal tube for providing breathing support to a patient, and vice versa.

The method may be implemented as a computer program having instructions, which, when executed by a computing device or computing system, cause the computing device or computing system to carry out the method for characterizing an inflatable cuff arranged around a tracheal tube for providing breathing support to a patient. A non-transitory storage medium may comprise a computer program product having instructions embodied thereon, wherein the computer program product, when executed by a computing device or system, causes the computing device or system to perform the method for characterizing an inflatable cuff arranged around a tracheal tube for providing breathing support to a patient.

### Description of drawings

The invention will in the following be described with reference to the accompanying drawings. The drawings are examples of embodiments and not limiting to the presently disclosed system for characterizing an inflatable cuff arranged around a tracheal tube for providing breathing support to a patient.
**Fig. 1** shows a schematic embodiment of the presently disclosed system for characterizing an inflatable cuff arranged around a tracheal tube.
**Fig. 2** shows a further schematic embodiment of the presently disclosed system for characterizing an inflatable cuff arranged around a tracheal tube.
**Fig. 3A-C** show examples of processes for extracting a pressure-volume relationship between the measured pressure and the estimated volume of gas or liquid in the inflatable cuff.
**Fig. 4A-C** show examples of pressure-volume relationships between the measured pressure and the estimated volume of gas or liquid in the inflatable cuff.
**Fig. 5A-C** show an example of a sequence of inflating and deflating the inflatable cuff to establish a pressure-volume relationships.
**Fig. 6A-C** show an example of a sequence of deflating and inflating the inflatable cuff to establish a pressure-volume relationships.
**Fig. 7** shows examples of pressure-volume relationships of the same inflatable cuff at different times.

### Detailed description

The present disclosure relates to a system for characterizing an inflatable cuff arranged around a tracheal tube for providing breathing support to a patient, the system comprising:
at least one flow unit, such as a pump, in fluid connection with a cuff conduit connected to the inflatable cuff, wherein the at least one flow unit is adapted to fill the inflatable cuff with gas or liquid;
at least one pressure sensor configured to measure a pressure in the inflatable cuff;
at least one flow sensor for measuring a flow of gas or liquid to and/or from the inflatable cuff;
a processing unit for estimating the volume of gas or liquid or a change of the volume of gas or liquid in the inflatable cuff based on measurements of the flow of gas or liquid to and/or from the inflatable cuff, wherein the processing unit is configured to:
   extract a pressure-volume relationship between the measured pressure and the estimated volume of gas or liquid or a change of the volume of gas or liquid in the inflatable cuff, and
   compare the pressure-volume relationship against a previously measured or a predetermined reference pressure-volume relationship to characterize the inflatable cuff.

By characterizing the inflatable cuff, in particular by analysing the pressure-volume relationship over time, conclusions can be made both regarding the inflatable cuff itself and the structure surrounding it. The characterization of the inflatable cuff may provide a measure of elasticity of the inflatable cuff and/or a measure of compliance. The system may be part of a ventilator or operate as an independent system.

The term 'tracheal tube' shall be construed broadly to comprise any suitable tube for maintaining a patient airway, including, but not limited to, endotracheal tubes and tracheostomy tubes. The inflatable cuff may accordingly be referred to as an inflatable endotracheal tube cuff or an inflatable tracheostomy tube cuff.

A 'processing unit' shall also be construed broadly to comprise any suitable means for processing and calculating data. The processing unit may be a local device, optionally integrated in a ventilator system, but may also be provided on a remote device, such as in a cloud computing device.

A flow sensor can be used to measure a flow rate of a gas or liquid. All types of flow sensor may be used to a flow of gas or liquid to and/or from the inflatable cuff according to the present disclosure. Based on the flow of gas or liquid over some time, a volume of gas or liquid or a change of the volume of gas or liquid in the inflatable cuff can be estimated. The presently disclosed system for characterizing an inflatable cuff is not limited to a specific flow sensor.

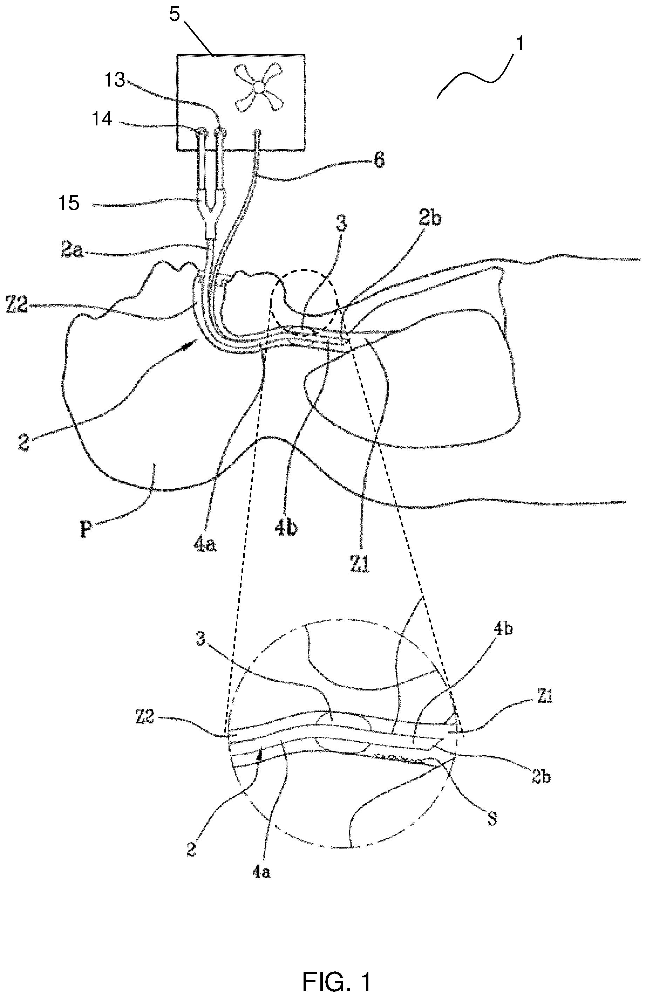

Fig. 1 shows a schematic embodiment of the general concept of intubation of a patient P with a tracheal tube 2 and an inflatable cuff 3. A system 1 for characterizing an inflatable cuff 3 arranged around a tracheal tube 3 for providing breathing support to a patient P is disclosed. As mentioned, the system 1 is connectable to or integrated in a ventilator for ventilation of the patient P. The tracheal tube 2 has a sidewall 2c and extends between a first end 2a and a second end 2b. The tracheal tube 2 is preferably made of PVC or the like, but may be made of any suitable material. The tracheal tube 2 has an associated inflatable cuff 3. The inflatable cuff 3 may be configured to seal the trachea of a patient P in order to isolate the second end 2b from the external environment. The inflatable cuff 3 may be an annular element externally fitted to the sidewall 2c of the tracheal tube 2. The inflatable cuff 3 divides the tracheal tube 2 into a first portion 4a and a second portion 4b. When positioned in the trachea, the inflatable cuff 3 also divides the trachea of the patient into an isolated zone Z1 and a non-sterile zone Z2.

The inflatable cuff 3 may have a substantially annular shape (donut-shaped). As would be recognized by a person skilled in the art, the inflatable cuff 3 may have any other suitable shape. Preferably, the inflatable cuff 3 is placed in fluid connection with at least a flow unit 5 through a conduit 6. The inflatable cuff 3 may have a chamber bound by an outer membrane. The outer membrane may be made of, for example, PVC less than 100 microns thick, or polyurethane having a thickness of less than 50 microns.

A flow unit 5 may also be connected to the tracheal tube 2. This may be a different flow unit than the one used for the inflatable cuff 3. It may be configured to generate a positive pressure and pause pressure and it may be are connected to the tracheal tube 2 through at least one connection mouth 13; 14 having an inlet mouth 13 and an outlet mouth 14, both being in fluid connection with the tracheal tube 2 through a two-input one-output connector 15.

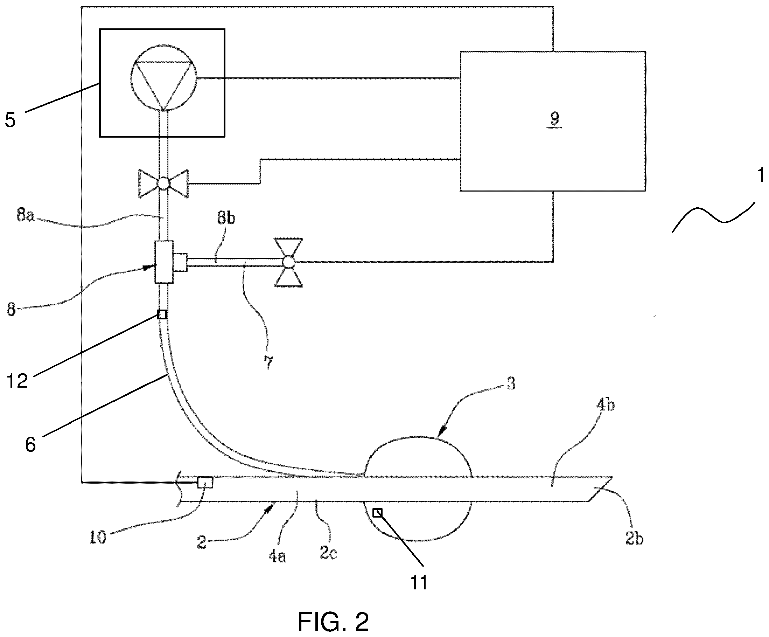

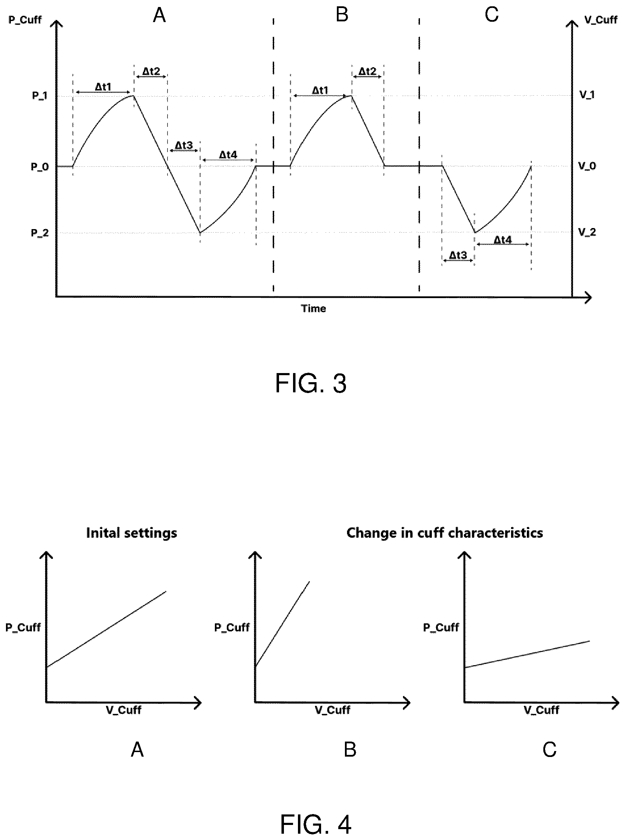

Fig. 2 shows a further schematic embodiment of the presently disclosed system 1 for characterizing an inflatable cuff 3 arranged around a tracheal tube 2. In fig. 3 a processing unit or control unit 9 controls the flow unit 5. The processing unit 9 may also estimate the volume of gas or liquid or a change of the volume of gas or liquid in the inflatable cuff based on measurements of the flow of gas or liquid to and/or from the inflatable cuff 3 based on a flow sensor 12. The flow sensor 12 may be placed at any suitable position. A pressure sensor 11 is configured to measure a pressure in the inflatable cuff 3. The pressure sensor 11 may be placed at any suitable position. The system 1 in the example of fig. has a venting conduit 7, which can be used for a parallel flow. The system 1 of. fig. 2 further comprises a fork 8 placed along the conduit 6.

According to an aspect of the present disclosure, the system and/or method comprises a step of inflating or deflating, or a combination of both, said inflatable cuff, either controlled by flow or pressure, in order to estimate the endotracheal tube cuff or tracheal tube cuff characteristics in an intubated patient.

Preferably, the step of generating a succession of inflating or deflating or a combination of both, said inflatable cuff, either controlled by flow or pressure, comprises steps of generating a cyclic succession of either an overpressure inside the inflatable cuff followed by a normal pressure setting inside the inflatable cuff, or an underpressure inside the inflatable cuff followed by a normal pressure setting inside the inflatable cuff, or a combination of both, in order to measure a pressure/ volume correlation inside the inflatable cuff.

The flow unit may be switchable between an inflation configuration, in which it inflates and/or maintains inflated the inflatable cuff, and a deflation configuration, wherein it deflates and/or maintains inflated the inflatable cuff, or a combination of inflation/deflation configuration.

In accordance with the presently disclosed system and method, the flow unit may be switched in a cyclical succession of first inflation configuration, deflation configuration or a combination of both, either manually or automatically.

The control module, which may comprise a processing unit, may be configured to control signals to the flow unit in order to activate or deactivate inflation or deflation. This may involve opening and closing of valves.

In one embodiment the system is configured to determine a degradation of the inflatable cuff based on the characterization of the inflatable cuff. A degradation of an inflatable cuff may be show as the inflatable cuff loses is resilience i.e. its ability to endure tribulation without cracking. A degradation may also be seen as a more elastic inflatable cuff. The processing unit is configured to compute or extract a measure of elasticity of the inflatable cuff based on the pressure-volume relationship.

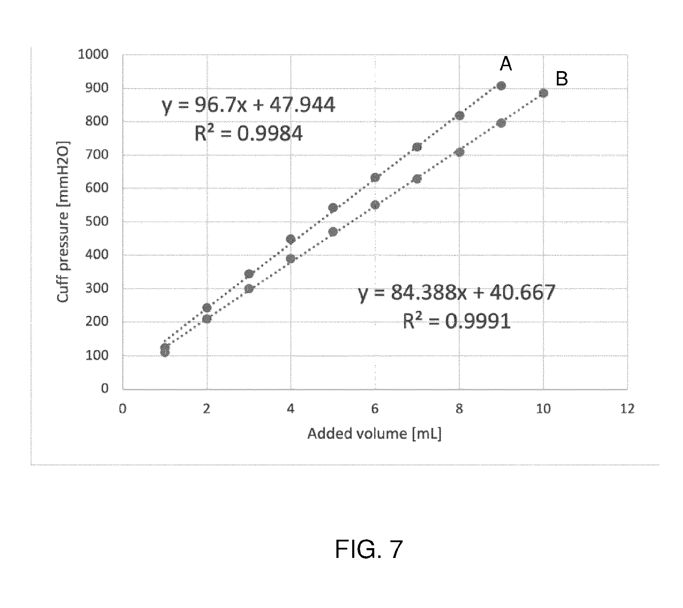

The pressure-volume relationship may describe a relationship between one or more given volume(s) and one or more corresponding pressure(s). The pressure-volume relationship describes a relationship between one or more given pressure(s) and one or more corresponding volumes(s). This can be illustrated, for example, by fig. 7, in which the volumes and pressures for two inflatable cuffs (or the same inflatable cuff at different points in time) A and B are shown. The pressure-volume relationship may be represented by a pressure-volume curve including measurements of a plurality of pressure levels and a plurality of volumes extracted from a plurality of measurements of flow of gas to and/or from the inflatable cuff. The pressure is provided on a first axis, such as a Y-axis, and the volume is provided on a second axis, such as an X-axis, of the curve. The pressure-volume relationship may be represented by a coefficient or formula converting at least one given volume to a corresponding pressure, or converting at least one given pressure to a corresponding volume. If it is a single pressure and volume it can be a single coefficient. If there are a number of measurements, the relationship can be expressed as an equation. A a higher coefficient may indicate that a higher pressure is required to obtain the given volume, and a lower coefficient may indicate that a lower pressure is required to obtain the given volume, or a higher coefficient may indicate that a smaller volume is required to obtain the given pressure, and a lower coefficient may indicate that a greater volume is required to obtain the given pressure. In the example of fig. 7 the inflatable cuff of scenario A a higher pressure is required to obtain the given volume compared to scenario B. Scenarios A and B could be for the same inflatable cuff. If B occurs later than A, it may be seen as B has become more elastic. If the pressure is provided on the Y-axis and the volume is provided on the X-axis, as in the example of fig. 7, a steeper curve may indicate a less elastic inflatable cuff and/or a higher coefficient, and a flatter curve indicates a more elastic inflatable cuff and/or a lower coefficient. In one embodiment, the processing unit is configured to categorize the inflatable cuff as worn out based on a change of the pressure-volume relationship over time. If the coefficient is less than a previously measured coefficient, the processing unit may be configured to categorize the inflatable cuff as worn out. Possibly the coefficient needs to be more than a predefined limit lower than previously measured coefficient in order for the inflatable cuff to be categorized as worn out.

The processing unit may additionally, or alternatively, be configured to identify a change in the structure around the cuff as a change in the pressure-volume relationship. The system may accordingly be configured to characterize a structure around the inflatable cuff based on the characterization of the inflatable cuff. The processing unit may be configured to identify a stiffer trachea of the patient as a result of an increase of the previously described coefficient, and/or configured to identify a softer trachea of the patient as a result of a decrease of the coefficient. In the example of fig. 7, if scenario B represents a first round of measurements and scenario A represents a subsequent second round of measurements, the higher coefficient of A may indicate that the trachea of the patient is stiffer.

In order to characterize the inflatable cuff, the system may be configured to collect the needed measurements. In one embodiment, the system may be configured to gradually fill the inflatable cuff with gas, or gradually empty the inflatable cuff, while the pressure sensor and the flow sensor, continuously, or at discrete points in time, measures the pressure in the inflatable cuff and the flow of gas to and/or from the inflatable cuff to generate a pressure-volume curve.

Fig. 3A-C show examples of processes for extracting a pressure-volume relationship between the measured pressure and the estimated volume of gas or liquid in the inflatable cuff.

In scenario A, the inflatable cuff is first inflated from a first pressure P_0 or first volume V_0 to a second pressure P_1 or a second volume V_1 in a second time interval Δt1. Preferably, both the pressure and the volume are measured, continuously, or at discrete points in time. The second pressure P_1 or second volume V_1 is then decreased to a third pressure P_2 or a third volume V_2 in a second and third time interval Δt2 and Δt3. The third pressure P_2 or third volume V_2 is then increased back to the first pressure P_0 or the first volume V_0 in a fourth time interval Δt4.

In scenario B, the inflatable cuff is first inflated from a first pressure P_0 or first volume V_0 to a second pressure P_1 or a second volume V_1 in a second time interval Δt1. The second pressure P_1 or second volume V_1 is then decreased to the first pressure P_0 or the first volume V_0 in a second time interval Δt2.

In scenario C, the inflatable cuff is first deflated from a first pressure P_0 or first volume V_0 to a third pressure P_2 or a third volume V_2 in a third time interval Δt3. The third pressure P_2 or third volume V_2 is then increased to the first pressure P_0 or the first volume V_0 in a fourth time interval Δt4.

Preferably, the time intervals are at least 0.01 seconds.

Preferably, the second pressure is at least 1 cmH2O higher than the first pressure. Preferably, the third pressure is at least 1 cmH2O lower than the first pressure.

Preferably, the second volume is at least 0.1 mL greater than the first volume. Preferably, the third volume is at least 0.1 mL smaller than the first volume.

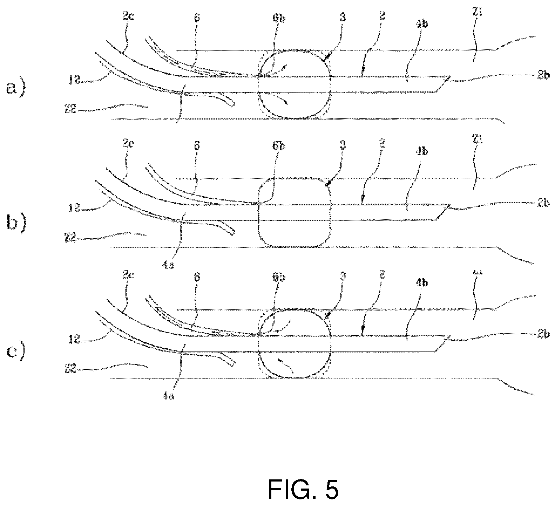

Fig. 5A-C show an example of a sequence of inflating and deflating the inflatable cuff to establish a pressure-volume relationships. The inflatable cuff starts in an initial state shown in fig. 5A. The pressure is then increased to a second state shown in fig. 5B. The pressure is then decreased to a third state shown in fig. 5C.

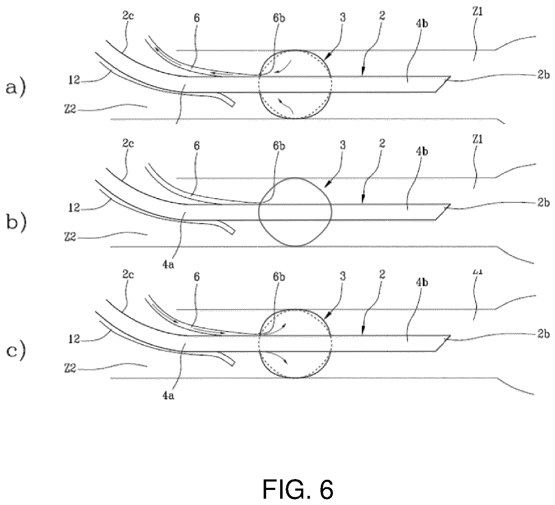

Fig. 6A-C show an example of a sequence of deflating and inflating the inflatable cuff to establish a pressure-volume relationships The inflatable cuff starts in an initial state shown in fig. 6A. The pressure is then decreased to a second state shown in fig. 6B. The pressure is then increased to a third state shown in fig. 6C.

Advantageously, in this way it is possible to characterize the tracheal tube in an intubated patient by correlating the measured pressure and volume of the inflatable as shown in fig. 4. Fig. 4A-C show examples of pressure-volume relationships between the measured pressure and the estimated volume of gas or liquid in the inflatable cuff.

Fig. 4A may be seen as an initial pressure-volume relationship between the pressure and volume of gas or liquid in the inflatable cuff. Figs. 4B and C may then be subsequent measurements of pressure-volume relationship between the pressure and volume of gas or liquid in the inflatable cuff. Fig. 4B may in this regard represent a stiffer trachea, whereas fig. 4C may represent a more elastic inflatable cuff.

The inflation volume of the inflatable cuff is to be determined according to type of patient and is therefore proportional to the size of the trachea and correlated to a target pressure to be generated in the inflatable cuff. However, the pressure inside the inflatable cuff may be determined independently of the volume. Indicatively, the pressure P_0 within the inflatable cuff, in order to prevent ischemia, is, preferably, between 20 and 30 cmH2O.

The disclosure further relates to a method for characterizing an inflatable cuff arranged around a tracheal tube for providing breathing support to a patient, the method comprising the steps of:
measuring a pressure in the inflatable cuff in the inflated configuration;
measuring a flow of gas to and/or from the inflatable cuff;
estimating the volume of gas in the inflatable cuff based on the measurements of the flow of gas to and/or from the inflatable cuff;
extracting a pressure-volume relationship between the measured pressure and the estimated volume of gas in the inflatable cuff, and
comparing the pressure-volume relationship against a previously measured or a predetermined reference pressure-volume relationship to characterize the inflatable cuff.

The method may comprise the steps of:
gradually filling the inflatable cuff with gas, and/or gradually emptying the inflatable cuff;
continuously, or at discrete points in time, measuring the pressure in the inflatable cuff and the flow of gas to and/or from the inflatable cuff to generate a pressure-volume curve.

## Claims

1. A system for characterizing an inflatable cuff arranged around a tracheal tube for providing breathing support to a patient, the system comprising:
at least one flow unit, such as a pump, in fluid connection with a cuff conduit connected to the inflatable cuff, wherein the at least one flow unit is adapted to fill the inflatable cuff with gas or liquid;
at least one pressure sensor configured to measure a pressure in the inflatable cuff;
at least one flow sensor for measuring a flow of gas or liquid to and/or from the inflatable cuff;
a processing unit for estimating the volume of gas or liquid or a change of the volume of gas or liquid in the inflatable cuff based on measurements of the flow of gas or liquid to and/or from the inflatable cuff, wherein the processing unit is configured to:
extract a pressure-volume relationship between the measured pressure and the estimated volume of gas or liquid or change of the volume of gas or liquid in the inflatable cuff, and
compare the pressure-volume relationship against a previously measured or a predetermined reference pressure-volume relationship to characterize the inflatable cuff.

2. The system for characterizing an inflatable cuff according to claim 1, wherein the system is configured to determine a degradation of the inflatable cuff based on the characterization of the inflatable cuff.

3. The system for characterizing an inflatable cuff according to any one of the preceding claims, wherein the system is further configured to characterize a structure around the inflatable cuff based on the characterization of the inflatable cuff.

4. The system for characterizing an inflatable cuff according to any one of the preceding claims, wherein the pressure-volume relationship describes a relationship between one or more given volume(s) and one or more corresponding pressure(s), or wherein the pressure-volume relationship describes a relationship between one or more given pressure(s) and one or more corresponding volumes(s).

5. The system for characterizing an inflatable cuff according to any one of the preceding claims, wherein the pressure-volume relationship is represented by a coefficient converting at least one given volume to a corresponding pressure, or converting at least one given pressure to a corresponding volume.

6. The system for characterizing an inflatable cuff according to claim 5, wherein a higher coefficient indicates that a higher pressure is required to obtain the given volume, and a lower coefficient indicates that a lower pressure is required to obtain the given volume, or a higher coefficient indicates that a smaller volume is required to obtain the given pressure, and a lower coefficient indicates that a greater volume is required to obtain the given pressure.

7. The system for characterizing an inflatable cuff according to any one of claims 4 to 6, wherein the processing unit is configured to categorize the inflatable cuff as worn out based on a change of the pressure-volume relationship over time, preferably, wherein, if the coefficient is less than a previously measured coefficient, the processing unit is configured to categorize the inflatable cuff as worn out .

8. The system for characterizing an inflatable cuff according to any one of the preceding claims, wherein the pressure-volume relationship is represented by a pressure-volume curve including measurements of a plurality of pressure levels and a plurality of volumes extracted from a plurality of measurements of flow of gas to and/or from the inflatable cuff.

9. The system for characterizing an inflatable cuff according to any one of the preceding claims, wherein the at least one flow unit is configured to gradually fill the inflatable cuff with gas, or gradually empty the inflatable cuff, while the pressure sensor and the flow sensor, continuously, or at discrete points in time, measures the pressure in the inflatable cuff and the flow of gas to and/or from the inflatable cuff to generate a pressure-volume curve.

10. The system for characterizing an inflatable cuff according to any one of the preceding claims, wherein the processing unit is configured to compute or extract a measure of elasticity of the inflatable cuff based on the pressure-volume relationship.

11. The system for characterizing an inflatable cuff according to any one of the preceding claims, wherein the processing unit is further configured to identify a change in the structure around the cuff as a change in the pressure-volume relationship.

12. The system for characterizing an inflatable cuff according to any one of claims 5 to 6, wherein the processing unit is further configured to identify a stiffer trachea and/or a smaller inner diameter of the trachea of the patient as a result of an increase of the coefficient, and/or wherein the processing unit is further configured to identify a softer trachea and/or a larger inner diameter of the trachea of the patient based on a change of the pressure-volume relationship over time.

13. The system for characterizing an inflatable cuff according to any one of claims 5 to 6, wherein the processing unit is further configured to identify a stiffer trachea and/or a smaller inner diameter of the trachea of the patient as a result of an increase of the coefficient, and/or wherein the processing unit is further configured to identify a softer trachea and/or a larger inner diameter of the trachea of the patient as a result of a decrease of the coefficient.

14. A method for characterizing an inflatable cuff arranged around a tracheal tube for providing breathing support to a patient, the method comprising the steps of:
measuring a pressure in the inflatable cuff in the inflated configuration;
measuring a flow of gas to and/or from the inflatable cuff;
estimating the volume of gas or a change of the volume of gas or liquid in the inflatable cuff based on the measurements of the flow of gas to and/or from the inflatable cuff;
extracting a pressure-volume relationship between the measured pressure and the estimated volume of gas in the inflatable cuff, and
comparing the pressure-volume relationship against a previously measured or a predetermined reference pressure-volume relationship to characterize the inflatable cuff.

15. A computer program having instructions which, when executed by a computing device or computing system, cause the computing device or computing system to carry out the method for characterizing an inflatable cuff arranged around a tracheal tube for providing breathing support to a patient according to claim 14.

## Patentansprüche

1. System zum Charakterisieren einer aufblasbaren Manschette, angeordnet um einen Trachealtubus zum Bereitstellen einer Atemunterstützung für einen Patienten, das System umfassend:
mindestens eine Strömungseinheit, wie eine Pumpe, in fluidischer Verbindung mit einer Manschettenleitung, verbunden mit der aufblasbaren Manschette, wobei die mindestens eine Strömungseinheit dazu eingerichtet ist, die aufblasbare Manschette mit Gas oder Flüssigkeit zu füllen;
mindestens einen Drucksensor, eingerichtet zum Messen eines Drucks in der aufblasbaren Manschette;
mindestens einen Strömungssensor zum Messen eines Strömungsflusses von Gas oder Flüssigkeit zu und/oder von der aufblasbaren Manschette;
eine Verarbeitungseinheit zum Abschätzen des Volumens von Gas oder Flüssigkeit oder einer Änderung des Volumens von Gas oder Flüssigkeit in der aufblasbaren Manschette basierend auf Messungen des Strömungsflusses von Gas oder Flüssigkeit zu und/oder von der aufblasbaren Manschette, wobei die Verarbeitungseinheit dazu eingerichtet ist:
eine Druck-Volumen-Beziehung zwischen dem gemessenen Druck und dem abgeschätzten Volumen von Gas oder Flüssigkeit oder einer Änderung des Volumens von Gas oder Flüssigkeit in der aufblasbaren Manschette zu extrahieren, und
die Druck-Volumen-Beziehung mit einer zuvor gemessenen oder einer vorbestimmten Referenz-Druck-Volumen-Beziehung zum Charakterisieren der aufblasbaren Manschette zu vergleichen.

2. System zum Charakterisieren einer aufblasbaren Manschette nach Anspruch 1, wobei das System dazu eingerichtet ist, einen Abbau der aufblasbaren Manschette basierend auf der Charakterisierung der aufblasbaren Manschette zu bestimmen.

3. System zum Charakterisieren einer aufblasbaren Manschette nach einem der vorhergehenden Ansprüche, wobei das System ferner dazu eingerichtet ist, eine Struktur um die aufblasbare Manschette basierend auf der Charakterisierung der aufblasbaren Manschette zu charakterisieren.

4. System zum Charakterisieren einer aufblasbaren Manschette nach einem der vorhergehenden Ansprüche, wobei die Druck-Volumen-Beziehung eine Beziehung zwischen einem oder mehreren gegebenen Volumen und einem oder mehreren entsprechenden Drücken beschreibt, oder wobei die Druck-Volumen-Beziehung eine Beziehung zwischen einem oder mehreren gegebenen Drücken und einem oder mehreren entsprechenden Volumen beschreibt.

5. System zum Charakterisieren einer aufblasbaren Manschette nach einem der vorhergehenden Ansprüche, wobei die Druck-Volumen-Beziehung durch einen Koeffizienten dargestellt ist, der mindestens ein gegebenes Volumen in einen entsprechenden Druck umwandelt, oder mindestens einen gegebenen Druck in ein entsprechendes Volumen umwandelt.

6. System zum Charakterisieren einer aufblasbaren Manschette nach Anspruch 5, wobei ein höherer Koeffizient anzeigt, dass ein höherer Druck erforderlich ist, um das gegebene Volumen zu erhalten, und ein niedrigerer Koeffizient anzeigt, dass ein niedrigerer Druck erforderlich ist, um das gegebene Volumen zu erhalten, oder ein höherer Koeffizient anzeigt, dass ein kleineres Volumen erforderlich ist, um den gegebenen Druck zu erhalten, und ein niedrigerer Koeffizient anzeigt, dass ein größeres Volumen erforderlich ist, um den gegebenen Druck zu erhalten.

7. System zum Charakterisieren einer aufblasbaren Manschette nach einem der Ansprüche 4 bis 6, wobei die Verarbeitungseinheit dazu eingerichtet ist, die aufblasbare Manschette basierend auf einer Änderung der Druck-Volumen-Beziehung über die Zeit als abgenutzt zu kategorisieren, vorzugsweise wobei, wenn der Koeffizient kleiner ist als ein zuvor gemessener Koeffizient, die Verarbeitungseinheit dazu eingerichtet ist, die aufblasbare Manschette als abgenutzt zu kategorisieren.

8. System zum Charakterisieren einer aufblasbaren Manschette nach einem der vorhergehenden Ansprüche, wobei die Druck-Volumen-Beziehung durch eine Druck-Volumen-Kurve dargestellt ist, die Messungen einer Vielzahl von Druckniveaus und einer Vielzahl von Volumen umfasst, die aus einer Vielzahl von Messungen des Strömungsflusses von Gas zu und/oder von der aufblasbaren Manschette extrahiert sind.

9. System zum Charakterisieren einer aufblasbaren Manschette nach einem der vorhergehenden Ansprüche, wobei die mindestens eine Strömungseinheit dazu eingerichtet ist, die aufblasbare Manschette schrittweise mit Gas zu füllen oder die aufblasbare Manschette schrittweise zu entleeren, während der Drucksensor und der Strömungssensor kontinuierlich oder zu diskreten Zeitpunkten den Druck in der aufblasbaren Manschette und den Strömungsfluss von Gas zu und/oder von der aufblasbaren Manschette messen, um eine Druck-Volumen-Kurve zu erzeugen.

10. System zum Charakterisieren einer aufblasbaren Manschette nach einem der vorhergehenden Ansprüche, wobei die Verarbeitungseinheit dazu eingerichtet ist, ein Maß der Elastizität der aufblasbaren Manschette basierend auf der Druck-Volumen-Beziehung zu berechnen oder zu extrahieren.

11. System zum Charakterisieren einer aufblasbaren Manschette nach einem der vorhergehenden Ansprüche, wobei die Verarbeitungseinheit ferner dazu eingerichtet ist, eine Änderung der Struktur um die Manschette als eine Änderung der Druck-Volumen-Beziehung zu identifizieren.

12. System zum Charakterisieren einer aufblasbaren Manschette nach einem der Ansprüche 5 bis 6, wobei die Verarbeitungseinheit ferner dazu eingerichtet ist, eine steifere Trachea und/oder einen kleineren Innendurchmesser der Trachea des Patienten als Ergebnis einer Erhöhung des Koeffizienten zu identifizieren, und/oder wobei die Verarbeitungseinheit ferner dazu eingerichtet ist, eine weichere Trachea und/oder einen größeren Innendurchmesser der Trachea des Patienten basierend auf einer Änderung der Druck-Volumen-Beziehung über die Zeit zu identifizieren.

13. System zum Charakterisieren einer aufblasbaren Manschette nach einem der Ansprüche 5 bis 6, wobei die Verarbeitungseinheit ferner dazu eingerichtet ist, eine steifere Trachea und/oder einen kleineren Innendurchmesser der Trachea des Patienten als Ergebnis einer Erhöhung des Koeffizienten zu identifizieren, und/oder wobei die Verarbeitungseinheit ferner dazu eingerichtet ist, eine weichere Trachea und/oder einen größeren Innendurchmesser der Trachea des Patienten als Ergebnis einer Verringerung des Koeffizienten zu identifizieren.

14. Verfahren zum Charakterisieren einer aufblasbaren Manschette, angeordnet um einen Trachealtubus zum Bereitstellen einer Atemunterstützung für einen Patienten, das Verfahren umfassend die Schritte von:
Messen eines Drucks in der aufblasbaren Manschette in der aufgeblasenen Konfiguration;
Messen eines Strömungsflusses von Gas zu und/oder von der aufblasbaren Manschette;
Abschätzen des Volumens von Gas oder einer Änderung des Volumens von Gas oder Flüssigkeit in der aufblasbaren Manschette basierend auf Messungen des Strömungsflusses von Gas zu und/oder von der aufblasbaren Manschette;
Extrahieren einer Druck-Volumen-Beziehung zwischen dem gemessenen Druck und dem abgeschätzten Volumen von Gas in der aufblasbaren Manschette, und
Vergleichen der Druck-Volumen-Beziehung mit einer zuvor gemessenen oder einer vorbestimmten Referenz-Druck-Volumen-Beziehung zum Charakterisieren der aufblasbaren Manschette.

15. Computerprogramm mit Anweisungen, die, wenn sie von einer Recheneinrichtung oder einem Rechensystem ausgeführt werden, die Recheneinrichtung oder das Rechensystem veranlassen, das Verfahren zum Charakterisieren einer aufblasbaren Manschette, angeordnet um einen Trachealtubus zum Bereitstellen einer Atemunterstützung für einen Patienten gemäß Anspruch 14, auszuführen.

## Revendications

1. Système pour caractériser un manchon gonflable disposé autour d'un tube trachéal pour fournir un support respiratoire à un patient, le système comprenant:
au moins une unité de débit, telle qu'une pompe, en connexion fluidique avec un conduit de manchon connecté au manchon gonflable, dans lequel ladite au moins une unité de débit est adaptée pour remplir le manchon gonflable avec un gaz ou un liquide;
au moins un capteur de pression configuré pour mesurer une pression dans le manchon gonflable;
au moins un capteur de débit pour mesurer un débit de gaz ou de liquide vers et/ou depuis le manchon gonflable;
une unité de traitement pour estimer le volume de gaz ou de liquide ou une variation du volume de gaz ou de liquide dans le manchon gonflable sur la base de mesures du débit de gaz ou de liquide vers et/ou depuis le manchon gonflable, dans lequel l'unité de traitement est configurée pour:
extraire une relation pression-volume entre la pression mesurée et le volume estimé de gaz ou de liquide ou la variation estimée du volume de gaz ou de liquide dans le manchon gonflable, et
comparer la relation pression-volume à une relation pression-volume de référence mesurée précédemment ou prédéterminée pour caractériser le manchon gonflable.

2. Système pour caractériser un manchon gonflable selon la revendication 1, dans lequel le système est configuré pour déterminer une dégradation du manchon gonflable sur la base de la caractérisation du manchon gonflable.

3. Système pour caractériser un manchon gonflable selon l'une quelconque des revendications précédentes, dans lequel le système est en outre configuré pour caractériser une structure autour du manchon gonflable sur la base de la caractérisation du manchon gonflable.

4. Système pour caractériser un manchon gonflable selon l'une quelconque des revendications précédentes, dans lequel la relation pression-volume décrit une relation entre un ou plusieurs volume(s) donné(s) et une ou plusieurs pression(s) correspondante(s), ou dans lequel la relation pression-volume décrit une relation entre une ou plusieurs pression(s) donnée(s) et un ou plusieurs volume(s) correspondant(s).

5. Système pour caractériser un manchon gonflable selon l'une quelconque des revendications précédentes, dans lequel la relation pression-volume est représentée par un coefficient convertissant au moins un volume donné en une pression correspondante, ou convertissant au moins une pression donnée en un volume correspondant.

6. Système pour caractériser un manchon gonflable selon la revendication 5, dans lequel un coefficient plus élevé indique qu'une pression plus élevée est requise pour obtenir le volume donné, et un coefficient plus faible indique qu'une pression plus faible est requise pour obtenir le volume donné, ou un coefficient plus élevé indique qu'un volume plus petit est requis pour obtenir la pression donnée, et un coefficient plus faible indique qu'un volume plus grand est requis pour obtenir la pression donnée.

7. Système pour caractériser un manchon gonflable selon l'une quelconque des revendications 4 à 6, dans lequel l'unité de traitement est configurée pour catégoriser le manchon gonflable comme usé sur la base d'un changement de la relation pression-volume au cours du temps, de préférence, dans lequel, si le coefficient est inférieur à un coefficient mesuré précédemment, l'unité de traitement est configurée pour catégoriser le manchon gonflable comme usé .

8. Système pour caractériser un manchon gonflable selon l'une quelconque des revendications précédentes, dans lequel la relation pression-volume est représentée par une courbe pression-volume comprenant des mesures d'une pluralité de niveaux de pression et d'une pluralité de volumes extraits d'une pluralité de mesures de débit de gaz vers et/ou depuis le manchon gonflable.

9. Système pour caractériser un manchon gonflable selon l'une quelconque des revendications précédentes, dans lequel ladite au moins une unité de débit est configurée pour remplir progressivement le manchon gonflable avec du gaz, ou vider progressivement le manchon gonflable, tandis que le capteur de pression et le capteur de débit, de manière continue, ou à des instants discrets, mesurent la pression dans le manchon gonflable et le débit de gaz vers et/ou depuis le manchon gonflable pour générer une courbe pression-volume.

10. Système pour caractériser un manchon gonflable selon l'une quelconque des revendications précédentes, dans lequel l'unité de traitement est configurée pour calculer ou extraire une mesure d'élasticité du manchon gonflable sur la base de la relation pression-volume.

11. Système pour caractériser un manchon gonflable selon l'une quelconque des revendications précédentes, dans lequel l'unité de traitement est en outre configurée pour identifier un changement dans la structure autour du manchon comme un changement de la relation pression-volume.

12. Système pour caractériser un manchon gonflable selon l'une quelconque des revendications 5 à 6, dans lequel l'unité de traitement est en outre configurée pour identifier une trachée plus rigide et/ou un diamètre interne plus petit de la trachée du patient comme résultat d'une augmentation du coefficient, et/ou dans lequel l'unité de traitement est en outre configurée pour identifier une trachée plus souple et/ou un diamètre interne plus grand de la trachée du patient sur la base d'un changement de la relation pression-volume au cours du temps.

13. Système pour caractériser un manchon gonflable selon l'une quelconque des revendications 5 à 6, dans lequel l'unité de traitement est en outre configurée pour identifier une trachée plus rigide et/or un diamètre interne plus petit de la trachée du patient comme résultat d'une augmentation du coefficient, et/ou dans lequel l'unité de traitement est en outre configurée pour identifier une trachée plus souple et/or un diamètre interne plus grand de la trachée du patient comme résultat d'une diminution du coefficient.

14. Procédé pour caractériser un manchon gonflable disposé autour d'un tube trachéal pour fournir un support respiratoire à un patient, le procédé comprenant les étapes de:
mesurer une pression dans le manchon gonflable dans la configuration gonflée;
mesurer un débit de gaz vers et/ou depuis le manchon gonflable;
estimer le volume de gaz ou une variation du volume de gaz ou de liquide dans le manchon gonflable sur la base des mesures du débit de gaz vers et/ou depuis le manchon gonflable;
extraire une relation pression-volume entre la pression mesurée et le volume estimé de gaz dans le manchon gonflable, et
comparer la relation pression-volume à une relation pression-volume de référence mesurée précédemment ou prédéterminée pour caractériser le manchon gonflable.

15. Programme informatique comportant des instructions qui, lorsqu'elles sont exécutées par un dispositif informatique ou un système informatique, amènent le dispositif informatique ou le système informatique à exécuter le procédé pour caractériser un manchon gonflable disposé autour d'un tube trachéal pour fournir un support respiratoire à un patient selon la revendication 14.
